# EUROPEAN PATENT APPLICATION

(11) **EP 1 477 484 A1**
(43) Date of publication of application: **17.11.2004**
(21) Application number: 04007653.1
(22) Date of filing: 26.02.2002
(51) Int. Cl.: C07D 319/06, C07D 213/22, C07D 405/14, C09B 23/02, G11B 7/24

(54) **Oxonol dye compound, optical information recording medium using the same, and optical information**

(30) Priority: 27.02.2001 JP 2001052293
(62) Divisional of application: 02004422.8
(71) Applicant: Fuji Photo Film Co., Ltd., Kanagawa (JP)
(72) Inventor: Morishima, Shinnichi, Minami-Ashigara-shi, Kanagawa (JP); Shibata, Michihiro, Odawara-shi, Kanagawa (JP)
(74) Representative: Klunker . Schmitt-Nilson . Hirsch

(57) **Abstract**

The present invention provides an oxonol dye compound represented by the following general formula (I): 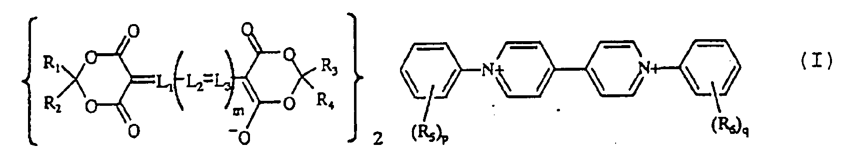 The symbols in the formula are defined in the description. Also provided are an optical information recording medium having a recording layer comprising the oxonol dye, and an optical information recording method using the recording medium.

## Description

### FIELD OF THE INVENTION

The present invention relates to a novel oxonol dye compound, an optical information recording medium using the oxonol dye compound, and a method for recording optical information. More particularly, the invention relates to an optical information recording medium such as CD-R or DVD-R, which is suitable for recording information using a near infrared laser beam or a visible laser beam, and a method for recording information using the optical information recording medium.

### BACKGROUND OF THE INVENTION

Information recording media (optical disks) which can record information only once with laser beams have hitherto been known. These information recording media are also called write once-type CDs (so-called CD-Rs), and have the advantage that the CDs can be provided in small amounts at reasonable prices and promptly, compared with the production of the conventional CDs. With the recent spread of personal computers, demand for such CDs have increased. The CD-R type information recording media have the typical structure that a transparent disk-shaped substrate is laminated with a recording layer comprising an organic dye, a light reflective layer comprising a metal such as gold or silver, and a resin protective layer in this order. Information is recorded on the optical disk by irradiating a near infrared laser beam (usually, a laser beam having a wavelength of about 780 nm) to locally deform the recording layer by heat generation. On the other hand, reading (reproduction) of the information is usually carried out by irradiating a laser beam having the same wavelength as the laser beam for recording to detect the difference in reflectance between a deformed site (recorded area) of the recording layer by heat generation and a non-deformed site (non-recorded area).

In recent years, information recording media higher in recording density have been desired. For increasing the recording density, it is effective to decrease the diameter of a laser beam irradiated. Further, it has been theoretically known that a laser beam having a shorter wavelength is effective for an increase in density, because the diameter of the laser beam can be decreased. Accordingly, optical disks for carrying out recording and reproduction using laser beams having wavelengths shorter than 780 nm which has hitherto been used have been developed. For example, an optical disk called a write once-type digital versatile disk (so-called DVD-R) has been proposed. This optical disk is produced so as to provide the structure that two disks each comprising a transparent disk-shaped substrate, having a diameter of 120 mm or 80 mm on which pre-grooves are formed at a track pitch of 0.7 µm to 0.8 µm which is narrower than 1.6 µm of the CD-R, having provided thereon a recording layer comprising a dye, and usually further a light reflective layer and a protective layer on the recording layer, or the disk and a disk-shaped protective substrate having a dimension approximately similar to that of the disk are adhered to each other using an adhesive, with rendering the recording layer(s) inside relative to the substrate(s). In the DVD-R, recording and reproduction are carried out by irradiation of a visible laser beam (usually, a laser beam having a wavelength ranging from 600 nm to 700 nm), and it is said that higher density recording than that of the CD-R is possible.

The DVD-R type information recording media can record information in an amount several times that of the CD-R type media. It is therefore desired that the probability of occurrence of errors in high-speed recording is small, particularly because of the necessity to rapidly process a large amount of information, not to mention that the DVD-R type media have high recording sensitivity. Further, the recording layers comprising the dyes are generally low in aging stability to heat or light, so that the development of recording layers which can maintain high performance to heat or light for a long period of time is desired.

Japanese Patent Laid-Open No. 2000-52658 (corresponding to US 6,225,024 B1) discloses an optical information recording medium comprising a substrate having provided thereon a recording layer comprising an oxonol dye in which an acidic nucleus has a meldrum acid structure. It is described that the use of the oxonol dye allows stable recording and reproduction characteristics to be maintained for a long period of time.

The present inventors have applied the oxonol dye described in Japanese Patent Laid-Open No. 2000-52658 to DVD-R type optical information recording media and studied their performance. The studies have proved that the DVD-R type optical information recording media containing the oxonol dye in recording layers are sufficiently satisfactory in recording and reproduction characteristics, but are liable to develop reproduction failure after the long-term storage thereof under the circumstances of high temperature and humidity, and insufficient in resistance against heat and humidity.

### SUMMARY OF THE INVENTION

Accordingly, an object of the invention is to provide an optical information recording medium having high recording characteristics and such high stability (particularly, high stability also under the circumstances of high temperature and humidity) that the recording characteristics can be sufficiently maintained for a long period of time.

Another object of the invention is to provide a novel oxonol dye compound which can be used in such an optical information recording medium.

Other objects and effects of the invention will become apparent from the following description.

The above-described objects of the present invention have been achieved by providing the followings:
(1) An oxonol dye compound represented by the following general formula (I): 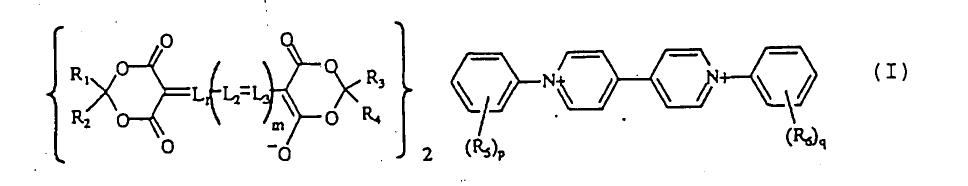 wherein R₁, R₂, R₃ and R₄ each independently represents an hydrogen atom, an alkyl group, an aryl group, an aralkyl group or a heterocyclic group; L₁, L₂ and L₃ each independently represents a methine group which may have a substituent group; m represents an integer of 0 to 3; R₅ and R₆ each represents an alkyl group having 4 to 8 carbon atoms, an aryl group having 6 to 12 carbon atoms, an aralkyl group having 7 to 12 carbon atoms, a chlorine atom, a bromine atom, a hydroxyl group, an acyl group having 7 to 12 carbon atoms, an aryloxy group having 6 to 12 carbon atoms or an amido group having 4 to 8 carbon atoms; p and q each represents an integer of 1 to 3; R₁ and R₂, and R₃ and R₄ may combine with each other to form a ring; when m is 2 or more, plural -L₂=L₃- groups may be the same or different; and when p and q are integers of 2 or more, plural R₅ and R₆ groups may be the same or different, respectively;
(2) An optical information recording medium comprising a substrate having thereon a recording layer capable of recording information by irradiation of a laser beam, wherein the recording layer contains an oxonol dye compound represented by general formula (I);
(3) The optical information recording medium according to the above (2), further comprising at least one of a reflective layer comprising a metal and a protective layer, provided on the recording layer.
(4) An optical information recording method comprising irradiating an optical information recording medium with a laser beam having a wavelength of 630 nm to 660 nm to record information thereon, wherein the optical information recording medium is according to the above (2) or (3).

### DETAILED DESCRIPTION OF THE INVENTION

The oxonol dye compounds, the optical information recording media and the optical information recording methods of the invention are described in detail below.

First, the oxonol dye compounds represented by the following general formula (I) are described. 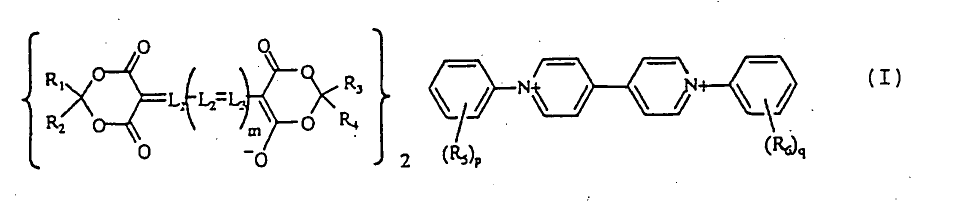 wherein R₁, R₂, R₃ and R₄ each independently represents an hydrogen atom, an alkyl group, an aryl group, an aralkyl group or a heterocyclic group; L₁, L₂ and L₃ each independently represents a methine group which may have a substituent group; m represents an integer of 0 to 3; R₅ and R₆ each represents an alkyl group having 4 to 8 carbon atoms, an aryl group having 6 to 12 carbon atoms, an aralkyl group having 7 to 12 carbon atoms, a chlorine atom, a bromine atom, a hydroxyl group, an acyl group having 7 to 12 carbon atoms, an aryloxy group having 6 to 12 carbon atoms or an amido group having 4 to 8 carbon atoms; p and q each represents an integer of 1 to 3; R₁ and R₂, and/or R₃ and R₄ may combine with each other to form a ring; when m is 2 or more, plural -L₂=L₃- groups may be the same or different; and when p and q are integers of 2 or more, plural R₅ and R₆ groups may be the same or different, respectively.

In general formula (I), R₁, R₂, R₃ and R₄ each independently represents a hydrogen atom, an alkyl group, an aryl group, an aralkyl group or a heterocyclic group.

The alkyl group is an alkyl group having 1 to 20 carbon atoms (e.g., methyl, ethyl, propyl, i-butyl, t-butyl, i-amyl, cyclopropyl, cyclohexyl), and may have a substituent group described below (extruding an alkyl group). Examples of the substituent groups (hereinafter referred to as "substituent group SUB") include an alkyl group having 1 to 20 carbon atoms (e.g., methyl, ethyl, propyl, carboxymethyl, ethoxycarbonylmethyl), an aralkyl group having 7 to 20 carbon atoms (e.g., benzyl, phenethyl), an alkoxyl group having 1 to 8 carbon atoms (e.g., methoxy, ethoxy), an aryl group having 6 to 20 carbon atoms (e.g., phenyl, naphthyl), an aryloxy group having 6 to 20 carbon atoms (e.g., phenoxy, naphthoxy), a heterocyclic group (e.g., pyridyl, pyrimidyl, pyridazyl, benzimidazolyl, benzothiazolyl, benzoxazolyl, 2-pyrrolidinone-1-yl, 2-piperidone-1-yl, 2,4-dioxyimidazolidine-3-yl, 2,4-dioxyoxazolidine-3-yl, succinimide, phthalimide, maleimide), a halogen atom (e.g., fluorine, chlorine, bromine, iodine), a carboxyl group, an alkoxycarbonyl group having 2 to 10 carbon atoms (e.g., methoxycarbonyl, ethoxycarbonyl), a cyano group, an acyl group having 2 to 10 carbon atoms (e.g., acetyl, pivaloyl), a carbamoyl group having 1 to 10 carbon atoms (e.g., carbamoyl, methylcarbamoyl, morpholinocarbamoyl), an amino group, a substituted amino group having 1 to 20 carbon atoms (e.g., dimethylamino, diethylamino, bis(methylsulfonylethyl)amino, N-ethyl-N'-sulfoethylamino), a sulfo group, a hydroxyl group, a nitro group, a sulfonamido group having 1 to 10 carbon atoms (e.g., methanesulfonamido), a ureido group having 1 to 10 carbon atoms (e.g., ureido, methylureido), a sulfonyl group having 1 to 10 carbon atoms (e.g., methanesulfonyl, ethanesulfonyl), a sulfinyl group having 1 to 10 carbon atoms (e.g., methanesulfinyl) and a sulfamoyl group having 0 to 10 carbon atoms (e.g., sulfamoyl, methanesulfamoyl). The carboxyl group and the sulfo group may be in the salt state.

The aryl group represented by R₁, R₂, R₃ and R₄ includes an aryl group having 6 to 20 carbon atoms (e.g., phenyl, naphthyl), and may have the above-defined substituent group SUB. The aralkyl group represented by R₁, R₂, R₃ and R₄ includes an aralkyl group having 7 to 20 carbon atoms (e.g., benzyl, phenethyl), and may have substituent group SUB. The heterocyclic group represented by R₁, R₂, R₃ and R₄ is a 5- or 6-membered saturated or unsaturated heterocyclic group comprising carbon atoms, nitrogen atoms, oxygen atoms or sulfur atoms, which include, for example, pyridyl, pyrimidyl, pyridazyl, piperidyl, triazyl, pyrrolyl, imidazolyl, triazolyl, furanyl, thiophenyl, thiazolyl, oxazolyl, isothiazolyl and isooxazolyl. The heterocyclic group may be a group obtained by the benzo cyclocondensation of them (e.g., quionlyl, benzimidazolyl, benzothiazolyl, benzoxazolyl), and may have, for example, substituent group SUB on the heterocycle.

As R₁, R₂, R₃ and R₄, preferred are a hydrogen atom, an alkyl group having 1 to 8 carbon atoms, an aryl group having 6 to 10 carbon atoms, an aralkyl group having 7 to 10 carbon atoms and a heterocyclic group having 4 to 10 carbon atoms. When R₁ and R₂, or R₃ and R₄ each represents an alkyl group, they may combine with each other to form a carbon ring (e.g., cyclopropyl, cyclobutyl, cyclopentyl, cyclohexyl, 2-methylcyclohexyl, cycloheptyl, cyclooctyl, adamantly) or a heterocycle (e.g., piperidyl, chromanyl, morpholyl), preferably a carbon ring having 3 to 10 carbon atoms or a heterocycle having 2 to 10 carbon atoms. In general formula (I), when R₁ and R₂ and/or R₃ and R₄ combine with each other to form a ring structure, excellent storage durability against heat and humidity is obtained.

L₁, L₂ and L₃ each independently represents an unsubstituted or substituted methine group. The substituent groups include substituent group SUB described above. Preferred examples of L₁, L₂ and L₃ include an unsubstituted methine group, a methine group substituted by an alkyl group having 1 to 5 carbon atoms, a methine group substituted by an aralkyl group having 7 to 10 carbon atoms, a methine group substituted by an aryl group having 6 to 10 carbon atoms, a methine group substituted by an unsubstituted or substituted heterocycle, and a , halogen-substituted methine group. m represents 0, 1, 2 or 3, and preferred is 1, 2 or 3. When m is 2 or more, plural L₂ and L₃ groups may be the same or different.

R₅ and R₆ each represents an unsubstituted or substituted alkyl group having 4 to 8 carbon atoms (e.g., butyl, pentyl), an aryl group having 6 to 12 carbon atoms (e.g., phenyl, naphthyl), an aralkyl group having 7 to 12 carbon atoms (e.g., benzyl), a chlorine atom, a bromine atom, a hydroxyl group, an acyl group having 7 to 12 carbon atoms (e.g., benzoyl), an aryloxy group having 6 to 12 carbon atoms (e.g., phenoxy) or an amido group having 4 to 8 carbon atoms. The substituent groups include substituent group SUB described above. As R₅ and R₆, particularly preferred are an aryl group having 6 to 12 carbon atoms, a hydroxyl group and an acyl group having 7 to 12 carbon atoms (e.g., benzoyl). p and q each represents an integer of 1 to 3. When p and q are each an integer of 2 or more, plural R₅ and R₆ groups may be the same or different.

Specific examples of the oxonol dye compounds represented by general formula (I) are enumerated below, but the dye compounds of the invention are not limited thereto.

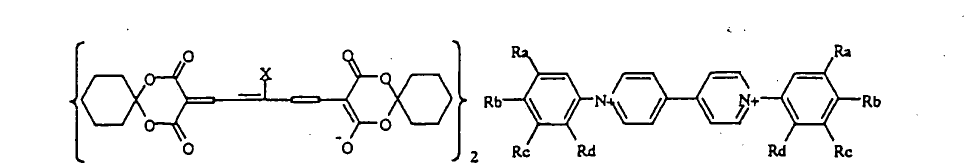

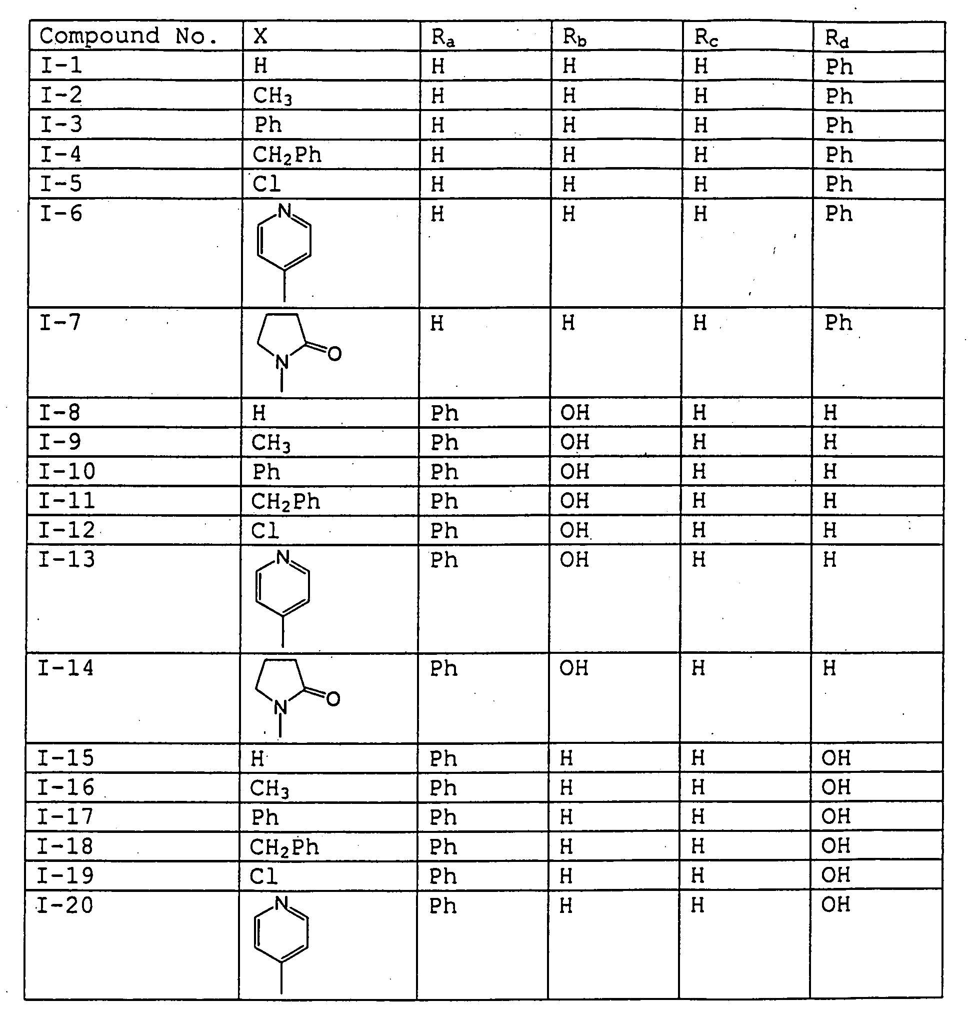

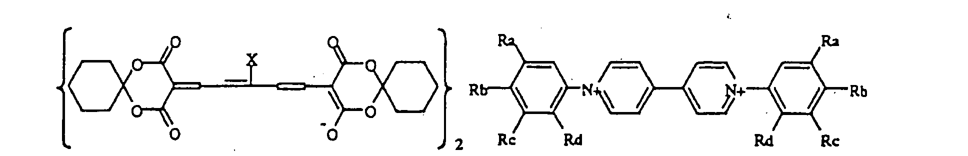

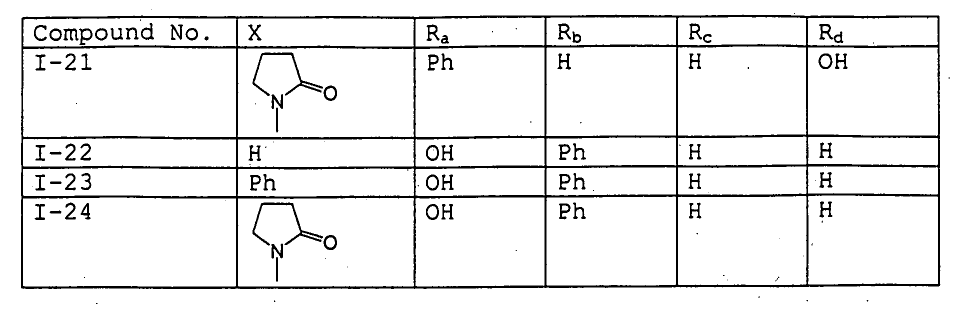

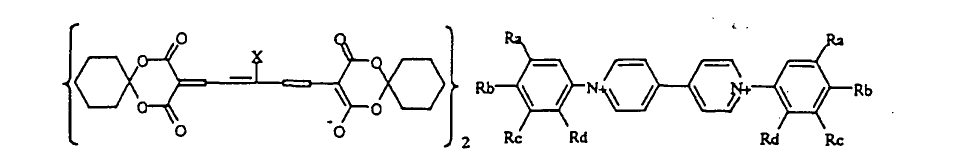

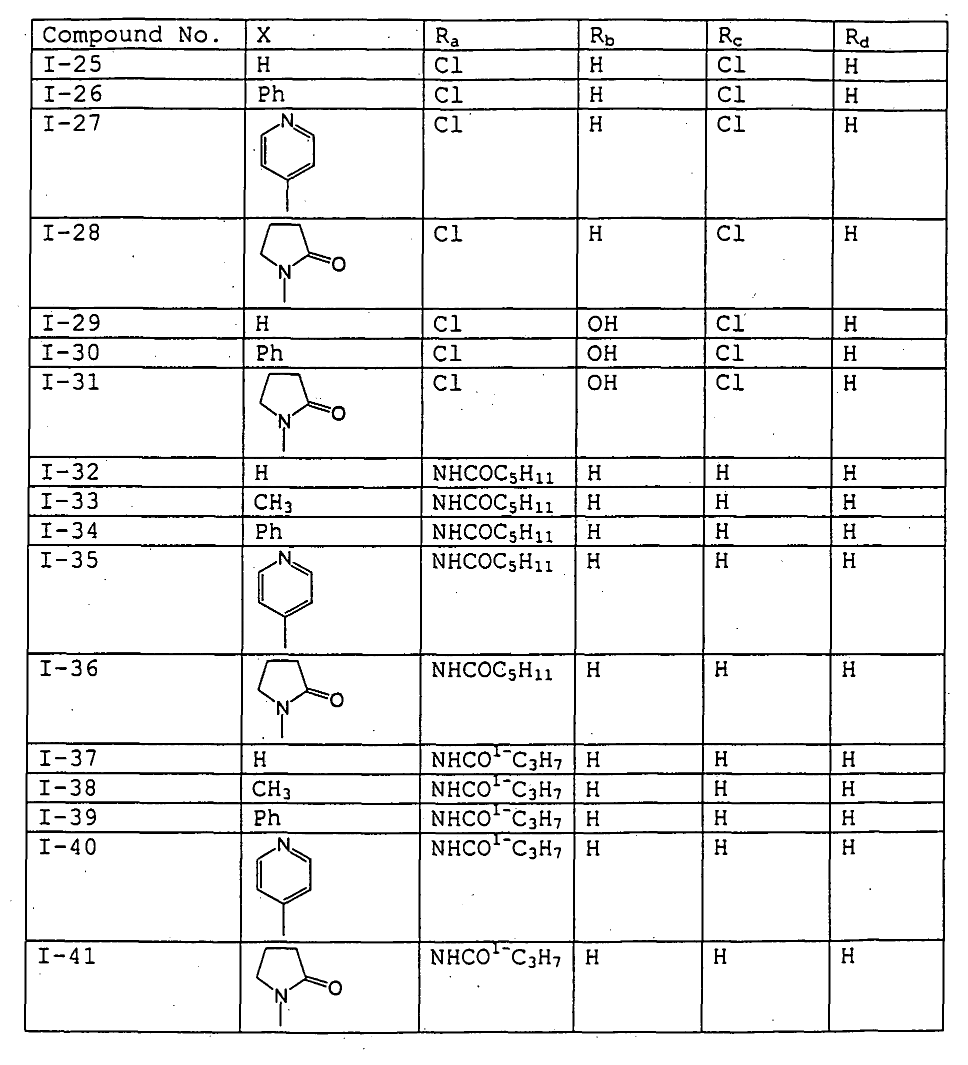

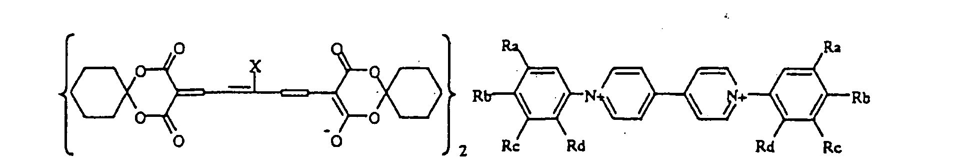

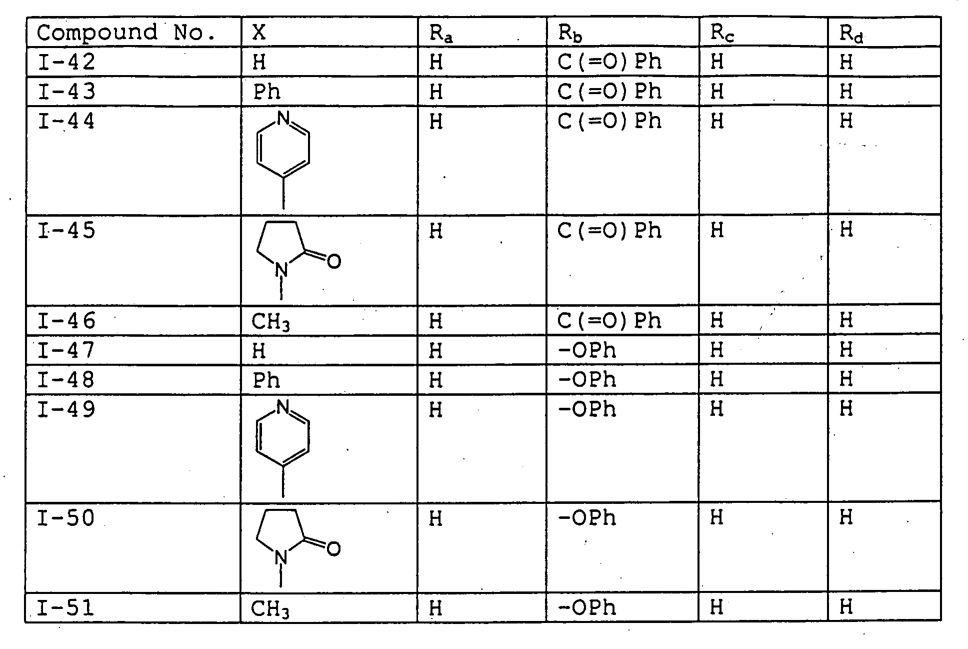

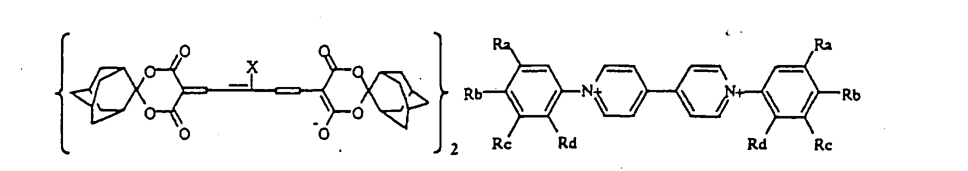

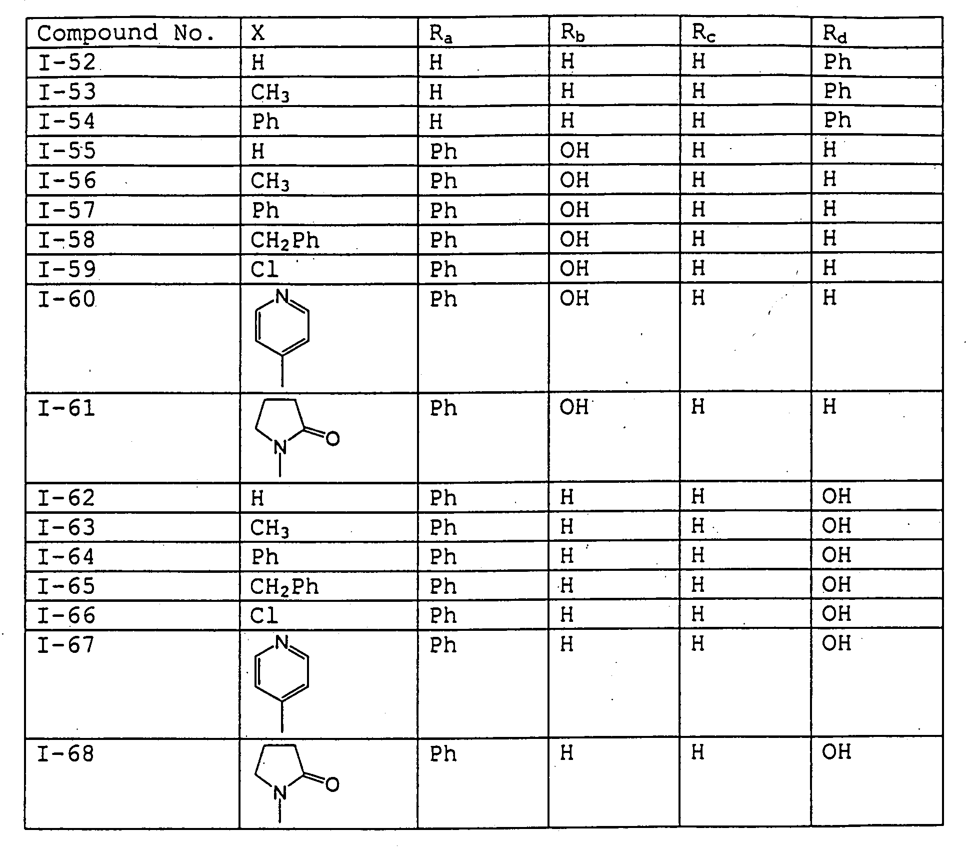

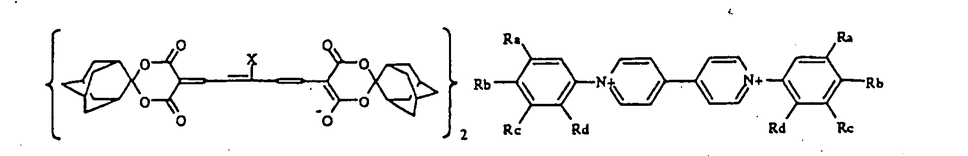

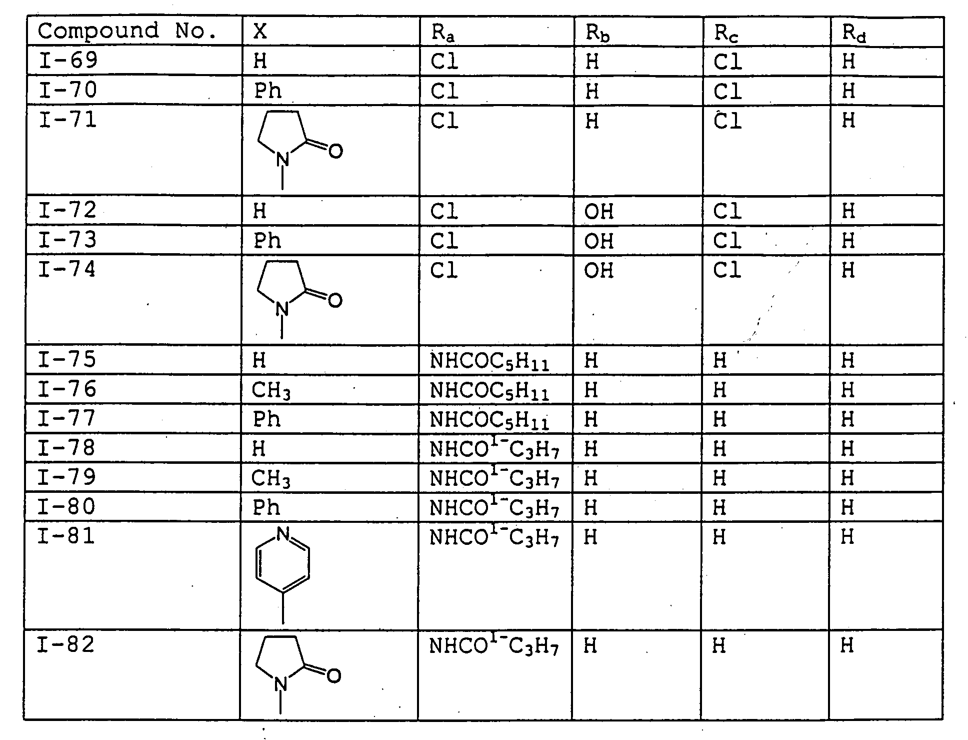

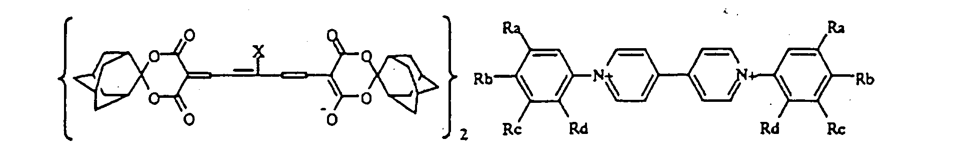

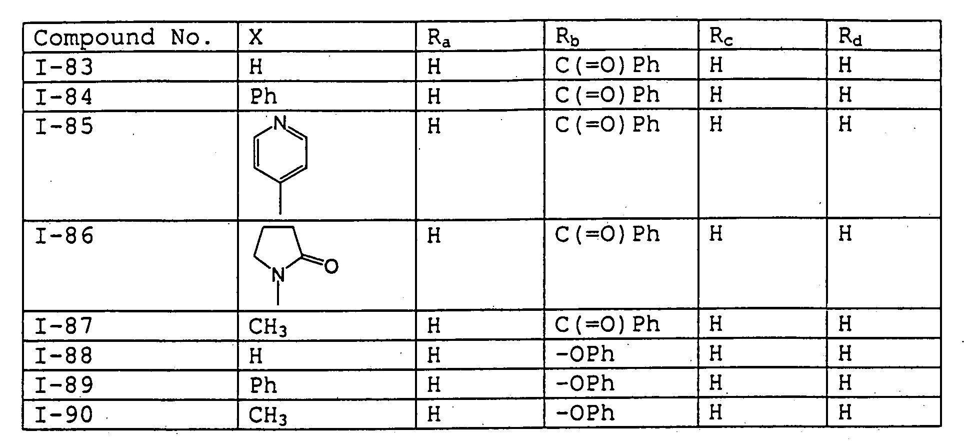

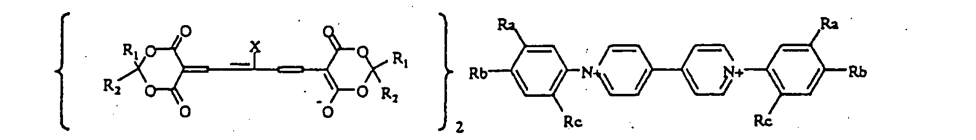

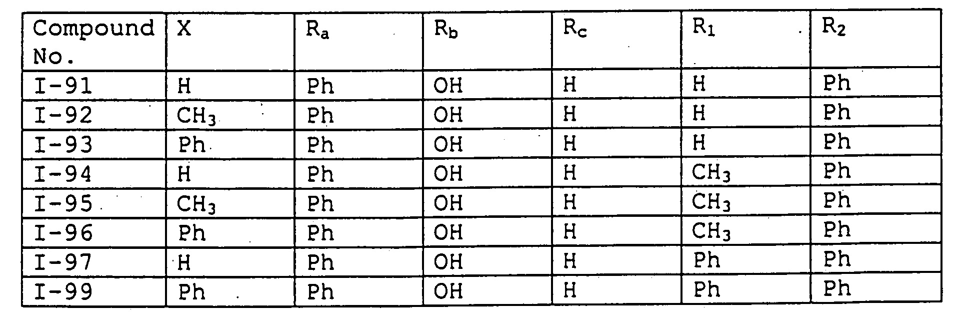

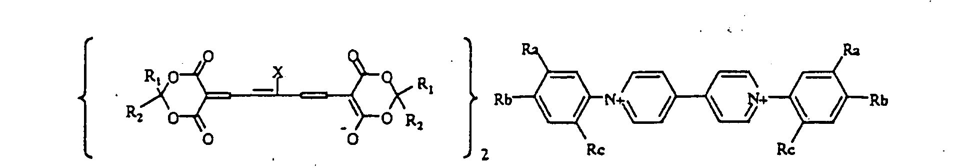

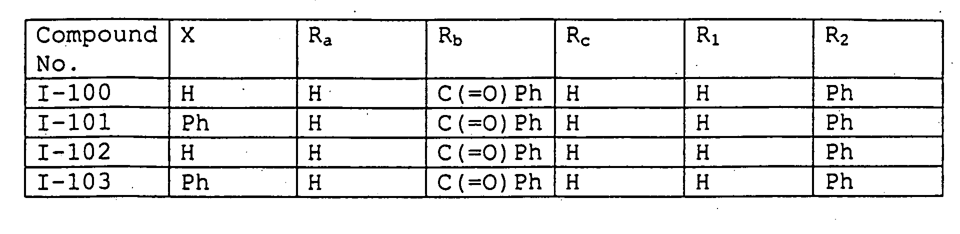

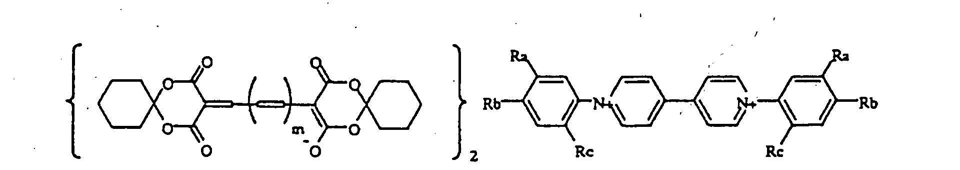

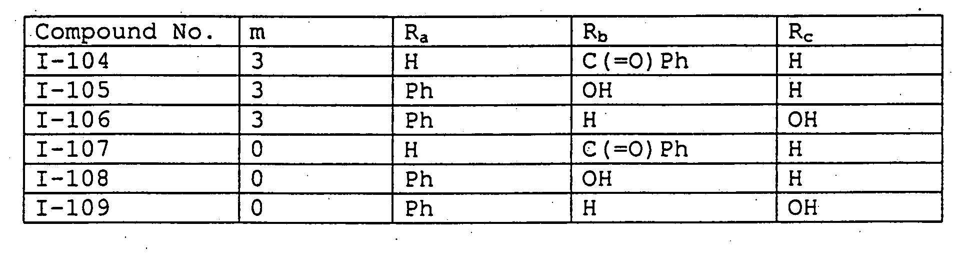

These compounds can be synthesized by methods based on synthesis methods described in Japanese Patent Laid-Open No. 2000-52658.

Synthesis examples of the oxonol dye compounds represented by general formula (I) will be described below.

### Synthesis Examples

First, synthesis examples of 4,4'-bipyridinium compounds are shown below.

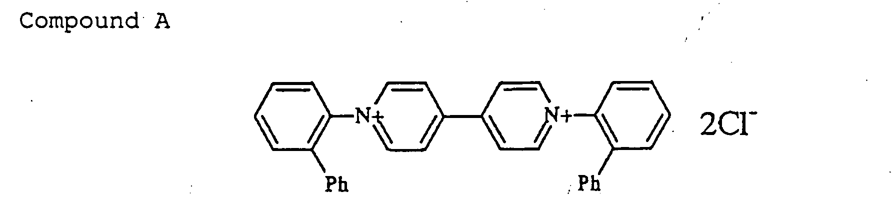

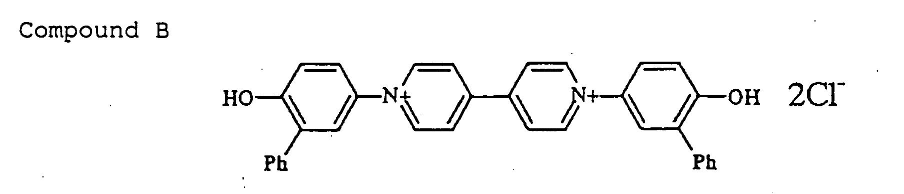

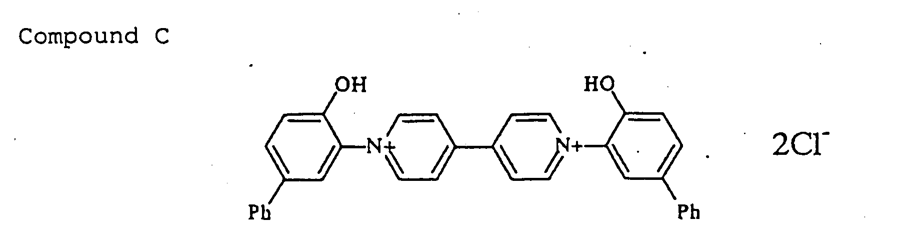

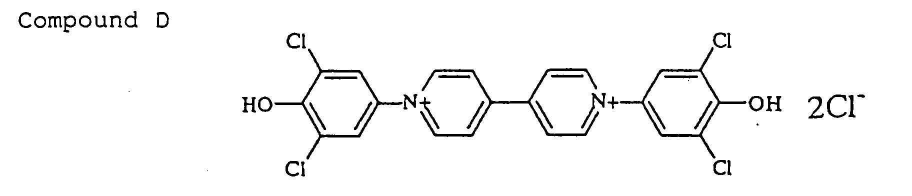

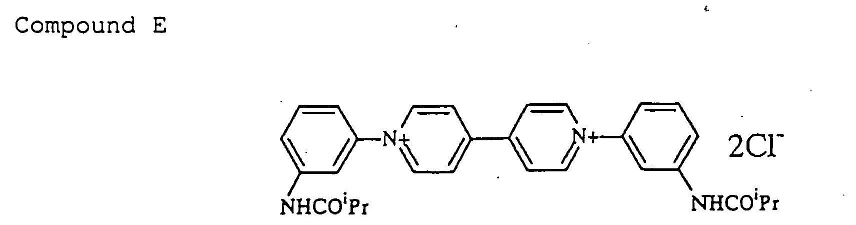

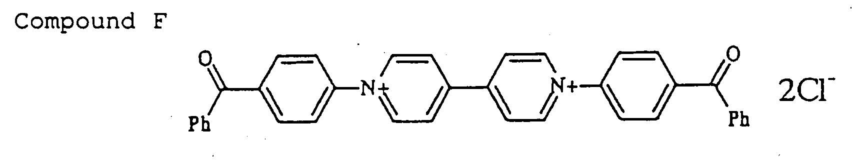

### Synthesis of Compound A

(1,1'-Bis(2,4-dinitrophenyl)-4,4'-bipyridinium dichloride (2.8 g, 5 mmol) and 2-aminobiphenyl (2.9 g, 0.017 mol) were dissolved in DMF (80 ml), followed by stirring at 95°C for 5 hours. After filtration of the reaction solution, the filtered product was washed with DMF (50 ml) to obtain 0.3 g of powdered yellow compound A. This corresponds to 11% of the theoretical yield.

¹H-NMR (DMSO-d6): 9.6 (d, 4H), 8.9 (d, 4H), 7.7-8.0 (m, 8H), 7.2-7.3 (m, 10H)

### Synthesis of Compound B

(1,1'-Bis(2,4-dinitrophenyl)-4,4'-bipyridinium dichloride (2.8 g, 5 mmol) and 4-amino-5-phenylphenol (3.2 g, 0.017 mol) were dissolved in DMF (80 ml), followed by stirring at 95°C for 6 hours. After filtration of the reaction solution, the filtered product was washed with DMF (50 ml) to obtain 3.0 g of powdered brown compound B.

¹H-NMR (DMSO-d6): 8.7-10.3 (broad, 8H), 7.6 (d, 3H), 7.3-7.5 (m, 5H)

### Synthesis of Compound C

(1,1'-Bis(2,4-dinitrophenyl)-4,4'-bipyridinium dichloride (16.1 g, 0.028 mol) and 2-amino-4-phenylphenol (12.8 g, 0.069 mol) were dissolved in DMF (100 ml), followed by stirring at 95°C for 6 hours. After filtration of the reaction solution, the filtered product was washed with DMF (50 ml) to obtain 15.5 g of powdered orange compound C. This corresponds to 98% of the theoretical yield.

¹H-NMR (DMSO-d6): 9.6 (d, 4H), 9.1 (d, 4H), 8.1 (s, 2H), 7.9 (d, 2H), 7.7 (d, 4H), 7.3-7.5 (m, 8H)

### Synthesis of Compound D

(1,1'-Bis(2,4-dinitrophenyl)-4,4'-bipyridinium dichloride (2.2 g, 4 mmol) and 4-amino-2,6-dichlorophenol (2.1 g, 0.012 mol) were dissolved in DMF (60 ml), followed by stirring at 95°C for 6 hours. After filtration of the reaction solution, the filtered product was washed with DMF (50 ml) to obtain 1.5 g of powdered orange compound D. This corresponds to 68% of the theoretical yield.

¹H-NMR (DMSO-d6): 9.6 (d, 4H), 9.1 (d, 4H), 8.1 (s, 4H)

### Synthesis of Compound E

(1,1'-Bis(2,4-dinitrophenyl)-4,4'-bipyridinium dichloride (2.8 g, 5 mmol) and 3-isobutanoylaminoaniline (3.1 g, 0.017 mol) were dissolved in DMF (60 ml), followed by stirring at 95°C for 6 hours. The reaction solution was added to ethyl acetate (500 ml), and stirred for 2 hours. Then, the reaction solution was subjected to filtration, and the filtered product was washed with ethyl acetate (50 ml) to obtain 2.7 g of powdered yellow compound E. This corresponds to 99% of the theoretical yield.

¹H-NMR (DMSO-d6): 11.0 (s, 2H), 9.7 (d, 4H), 9.1 (d, 4H), 8.5 (s, 2H), 7.9 (m, 2H), 7.5-7.7 (m, 4H), 2.8 (m, 2H), 1.2 (d, 12H)

### Synthesis of Compound F

(1,1'-Bis(2,4-dinitrophenyl)-4,4'-bipyridinium dichloride (7.8 g, 0.014 mol) and 4-aminobenzophenone (6.6 g, 0.043 mol) were dissolved in DMF (100 ml), followed by stirring at 95°C for 6 hours. Then, the reaction solution was subjected to filtration, and the filtered product washed with DMF (50 ml) to obtain 4.7 g of powdered white compound F. This corresponds to 57% of the theoretical yield.

¹H-NMR (MeOD): 8.8-9.8 (broad, 8H), 8.1 (broad, 8H), 7.8 (d, 4H), 7.7 (dd, 2H), 7.5 (dd, 4)

Then, synthesis examples of the oxonol dye compounds are shown below.

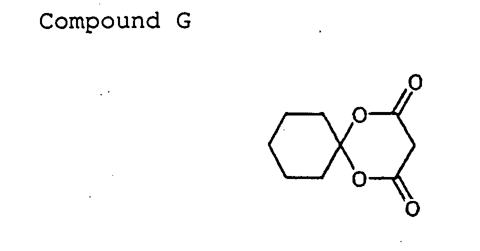

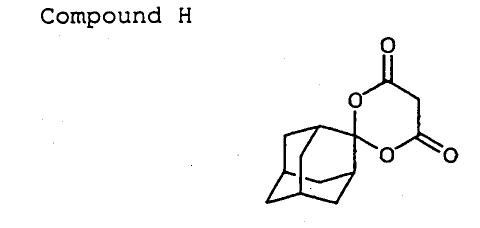

### Synthesis of Compound G

Malonic acid (41.6 g, 0.40 mol) and cyclohexanone (43.2 g, 0.44 mol) were dissolved in acetic anhydride (48 ml), and then, sulfuric acid (1.2 ml) was added dropwise thereto. After stirring at room temperature for 8 hours, water (800 ml) and hexane (600 ml) were in turn poured into the resulting solution, followed by stirring at room temperature for 1 hour. The resulting crystals were filtered and washed with water (100 ml) and hexane (100 ml) to obtain 50 g of powdered white compound G. This corresponds to 68% of the theoretical yield.

¹H-NMR (CDCl₃) : 3.6 (s, 2H), 1.9 (m, 4H), 1.6-1.8 (m, 4H), 1.5 (m, 2H)

### Synthesis of Compound H

Malonic acid (10.4 g, 0.10 mol) and adamantanone (16.5 g, 0.11 mol) were dissolved in acetic anhydride (13 ml), and then, sulfuric acid (0.2 ml) was added dropwise thereto. After stirring at room temperature for 8 hours, water (400 ml) and hexane (200 ml) were in turn poured into the resulting solution, followed by stirring at room temperature for 1 hour. The resulting crystals were filtered and washed with water (100 ml) and hexane (100 ml) to obtain 22 g of powdered white compound H. This corresponds to 93% of the theoretical yield.

¹H-NMR (CDCl₃): 3.6 (s, 2H), 2.0-2.3 (m, 6H), 1.6-2.0 (m, 8H)

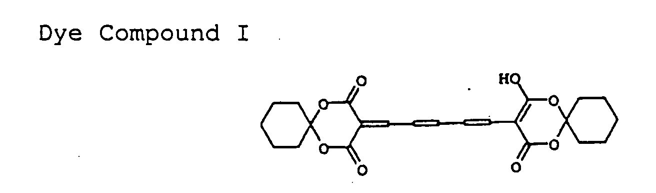

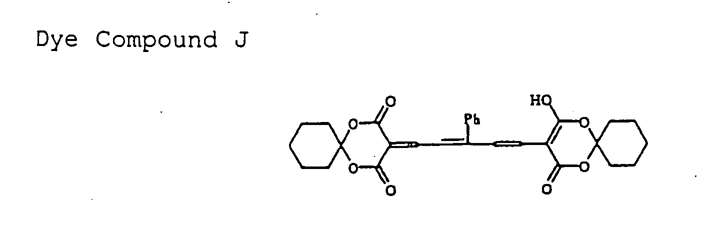

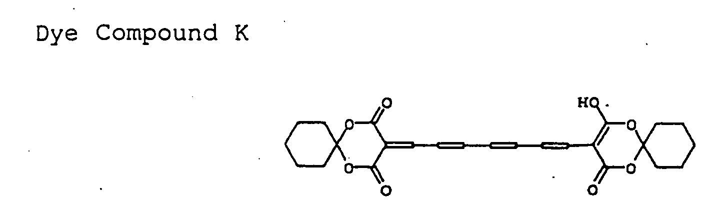

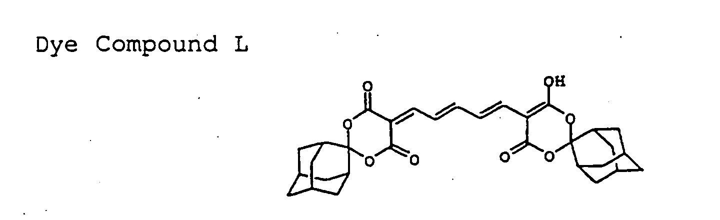

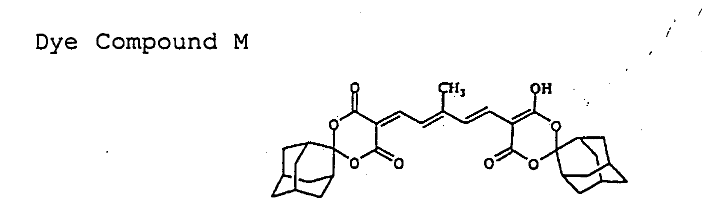

### Synthesis of Dye Compound I

Compound G (32 g, 0.17 mol) and N,N'-1,3-pentadiene-1-yl-5-ylidenedianiline hydrochloride (21 g, 0.074 mol) were dissolved in DMF (150 ml), and then, triethylamine (36 ml) was added dropwise thereto. After stirring at room temperature for 6 hours, extraction was conducted with saturated brine (400 ml) and ethyl acetate (400 ml). Crystals precipitated in the extraction process were filtered and washed with water (100 ml) and ethyl acetate (100 ml) to obtain 31 g of powdered deep red dye compound I. This corresponds to 97% of the theoretical yield.

¹H-NMR (DMSO-d6): 7.8 (d, 2H), 7.5 (dd, 1H), 7.1 (d, 2H), 1. 9 (m, 8H), 1.5 (m, 8H), 1.4 (m, 4H)

### Synthesis of Dye Compound J

Compound G (39 g, 0.21 mol) and compound N described below (34 g, 0.10 mol) were dissolved in ethyl acetate (200 ml), and then, triethylamine (47 ml) was added dropwise thereto. After heating reflux for 3 hours, ethyl acetate (100 ml), hexane (100 ml), water (200 ml) and concentrated hydrochloric acid (28 ml) were added, followed by stirring at room temperature for 2 hours. Crystals precipitated were filtered and washed with water (100 ml) and ethyl acetate (100 ml) to obtain 68 g of powdered deep blue dye compound J.

¹H-NMR (DMSO-d6): 7.5 (m, 6H), 7.0-7.2 (m, 3H), 1.9 (m, 8H), 1.5 (m, 8H), 1.4 (m, 4H)

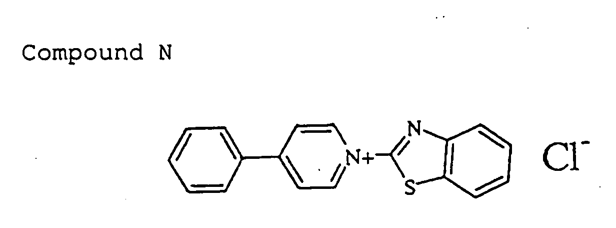

### Synthesis of Dye Compound K

Compound G (7.4 g, 0.040 mol) and N,N'-heptadiene-1-yl-7-ylidenedianiline hydrochloride (5.5 g, 0.020 mol) were dissolved in DMF (50 ml), and then, triethylamine (5.6 ml) and acetic anhydride (3.8 ml) were added dropwise thereto. After stirring at room temperature for 1 hour, extraction was conducted with saturated brine (400 ml) and ethyl acetate (400 ml). Crystals precipitated in the extraction process were filtered and washed with water (100 ml) and ethyl acetate (100 ml) to obtain 1.4 g of powdered blue dye compound K. This corresponds to 15%.of the theoretical yield.

¹H-NMR (DMSO-d6): 7.6 (d, 2H), 7.1-7.4 (m, 4H), 6.3 (dd, 1H), 1.8 (m, 8H), 1.5 (m, 8H), 1.4 (m, 4H)

### Synthesis of Dye Compound L

Compound H (5.2.g, 0.022 mol) and N,N'-1,3-pentadiene-1-yl-5-ylidenedianiline hydrochloride (2.8 g, 0.010 mol) were dissolved in DMF (30 ml), and then, triethylamine (4.5 ml) was added dropwise thereto. After stirring at room temperature for 5 hours, extraction was conducted with saturated brine (400 ml) and ethyl acetate (400 ml). Crystals precipitated in the extraction process were filtered and washed with water (100 ml) and ethyl acetate (100 ml) to obtain 4.5 g of powdered deep red dye compound L. This corresponds to 84% of the theoretical yield.

¹H-NMR (DMSO-d6): 7.6 (d, 2H), 7.5 (dd, 1H), 7.1 (dd, 2H), 2.1 (s, 4H), 1.9 (m, 8H), 1.8 (s, 4H), 1.6 (m, 12H)

### Synthesis of Dye Compound M

Compound H (23.6 g, 0.10 mol) and compound O described below (17.1 g, 0.050 mol) were dissolved in DMF (100 ml), and then, triethylamine (21 ml) was added dropwise thereto. After stirring at room temperature for 5 hours, extraction was conducted with saturated brine (400 ml) and ethyl acetate (400 ml). Crystals precipitated in the extraction process were filtered and washed with water (100 ml) and ethyl acetate (100 ml) to obtain 23.4 g of powdered deep red dye compound M. This corresponds to 85% of the theoretical yield.

¹H-NMR (DMSO-d6): 7.8 (d, 2H), 7.7 (d, 2H), 2.1 (s, 7H), 1.9 (m, 8H), 1.8 (s, 4H), 1.6 (m, 12H)

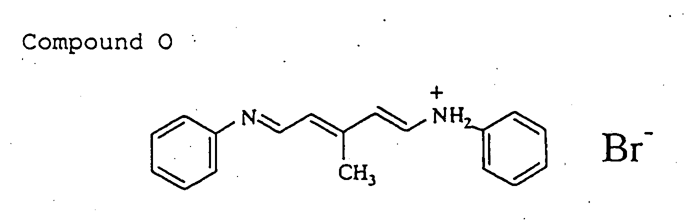

### Synthesis of Dye Compound (I-8)

Dye compound I (12.9 g, 0.030 mol) was dissolved in methanol (150 ml), and then, compound B (8.5 g, 0.015 mol) was added thereto. After stirring at room temperature for 5 hours, the resulting solution was subjected to filtration, and the filtered product was washed with methanol (100 ml) to obtain 17.0 g of powdered green dye compound (I-8). This corresponds to 84% of the theoretical yield.

¹H-NMR (DMSO-d6): 10.7 (s, 2H), 9.7 (d, 4H), 9.0 (d, 4H), 7.0-8.0 (m, 26H), 1.9 (m, 16H), 1.5 (m, 16H), 1.4 (m, 8H)

### Synthesis of Dye Compound (I-10)

Dye compound J (8.5 g, 0.017 mol) was dissolved in methanol (150 ml), and then, compound B (4.7 g, 8.3 mmol) was added thereto. After stirring at room temperature for 5 hours, the resulting solution was subjected to filtration, and the filtered product was washed with methanol (100 ml) to obtain 10.1 g of powdered green dye compound (I-10). This corresponds to 79% of the theoretical yield.

¹H-NMR (DMSO-d6): 10.7 (s, 2H), 9.7 (d, 4H), 9.0 (d, 4H), 7.7-7.9 (m, 6H), 7.1-7.6 (m, 26H), 1.9 (m, 16H), 1.5 (m, 16H), 1.4 (m, 8H)

### Synthesis of Dye Compound (I-15)

Dye compound I (12.9 g, 0.030 mol) was dissolved in methanol (400 ml), and then, compound C (8.5 g, 0.015 mol) was added thereto. After stirring at room temperature for 5 hours, the resulting solution was subjected to filtration, and the filtered product was washed with methanol (100 ml) to obtain 19.3 g of powdered green dye compound (I-15). This corresponds to 95% of the theoretical yield.

¹H-NMR (DMSO-d6): 9.7 (d, 4H), 9.0 (d, 4H), 8.0 (s, 2H), 7.7-7.9 (d, 2H), 7.5-7.7 (m, 8H), 7.3-7.5 (m, 10H), 7.1 (dd, 4H), 1.9 (m, 16H), 1.5 (m, 16H), 1.4 (m, 8H)

### Synthesis of Dye Compound (I-52)

Dye compound L (0.32 g, 0.6 mmol) was dissolved in methanol (10 ml), and then, compound A (0.16 g, 0.3 mmol) was added thereto. After stirring at room temperature for 3 hours, the resulting solution was subjected to filtration, and the filtered product was washed with methanol to obtain 0.33 g of dye compound (I-52) having a golden luster. This corresponds to 72% of the theoretical yield.

¹H-NMR (DMSO-d6): 9.6 (d, 4H), 8.9 (d, 4H), 7.1-7.9 (m, 28H), 2.1 (s, 8H), 1.9 (m, 16H), 1.8 (s, 8H), 1.6 (m, 24H)

### Synthesis of Dye Compound (I-72)

Dye compound L (0.53 g, 1.0 mmol) was dissolved in methanol (10 ml) containing concentrated hydrochloric acid (1 ml), and then, compound D (0.33 g, 0.6 mmol) was added thereto. After stirring at room temperature for 3 hours, the resulting solution was subjected to filtration, and the filtered product was washed with methanol to obtain 0.35 g of green crystals of dye compound (I-72). This corresponds to 45% of the theoretical yield.

¹H-NMR (DMSO-d6): 11.4 (s, 2H), 9.7 (d, 4H), 9.1 (d, 4H), 8.1 (s, 4H), 7.6 (d, 4H), 7.5 (dd, 2H), 7.1 (dd, 4H), 2.1 (s, 8H), 1.9 (m, 16H), 1.8 (s, 8H), 1.6 (m, 24H)

### Synthesis of Dye Compound (I-78)

Dye compound L (10.7 g, 0.0 20 mol) was dissolved in methanol (100 ml), and then, compound E (6.6 g, 0.012 mol) was added thereto. After stirring at room temperature for 2 hours, the resulting solution was subjected to filtration, and the filtered product was washed with methanol to obtain 12.2 g of green crystals of dye compound (I-78). This corresponds to 79% of the theoretical yield.

¹H-NMR (DMSO-d6): 10.4 (s, 2H), 9.7 (d, 4H), 9.1 (d, 4H), 8.5 (s, 2H), 7.6-7.8 (m, 10H), 7.6 (dd, 2H), 7.1 (dd, 4H), 2.7 (tt, 2H), 2.1 (s, 8H), 1.9 (m, 16H), 1.8 (s, 8H), 1.6 (m, 24H), 1.2 (d, 12H)

### Synthesis of Dye Compound (I-87)

Dye compound M (0.44 g, 0.8 mmol) was dissolved in methanol (100 ml), and then, compound F (0.24 g, 0.4 mmol ) was added thereto. After stirring at room temperature for 2 hours, the resulting solution was subjected to filtration; and the filtered product was washed with methanol to obtain 0.37 g of dye compound (I-87) having a golden luster. This corresponds to 57% of the theoretical yield.

¹H-NMR (DMSO-d6): 9.8 (d, 4H), 9.2 (d, 4H), 8.1 (m, 8H), 7.9 (d, 4H), 7.7-7.8 (m, 6H), 7.6 (dd, 4H), 7.2 (d, 4H), 2.1 (s, 14H), 1.9 (m, 16H), 1.8 (s, 8H), 1.6 (m, 24H)

### Synthesis of Dye Compound (I-104)

Dye compound K (0.92 g, 2.0 mmol) was dissolved in methanol (100 ml), and then, compound F (0.71 g, 1.2 mmol) was added thereto. After stirring at room temperature for 4 hours, the resulting solution was subjected to filtration, and the filtered product was washed with methanol to obtain 1.1 g of green crystals of dye compound (I-104). This corresponds to 77% of the theoretical yield.

¹H-NMR (DMSO-d6): 9.8 (d, 4H), 9.2 (d, 4H), 8.1 (m, 8H), 7.8-7.9 (m, 6H), 7.7 (dd, 4H), 7.5 (d, 4H), 7.0-7.3 (m, 8H), 6.2 (dd, 2H), 1.9 (m, 16H), 1.5 (m, 16H), 1.4 (m, 8H)

Then, the optical information recording media of the invention will be described. The optical information recording medium of the invention comprises a substrate having provided thereon a recording layer containing at least on oxonol dye compound represented by general formula (I). The oxonol dye compound for use in the invention can be appropriately selected depending on the wavelength of light used for recording and reproduction. For example, when recording is carried out with a semiconductor laser beam having a center wavelength of 780 nm which is used for the CD-R recording, dye compounds in which m is 3 in general formula (I) can be used, and when recording is carried out with a laser beam having a wavelength ranging from 630 nm to 660 nm which is used for the DVD-R recording, dye compounds in which m is 2 can be used. Further, when recording is carried out with a laser beam having a wavelength 550 nm or less, dye compounds in which m is 0 or 1 (preferably 0) can be used.

The dye compounds according to the invention may be used either singly or as a combination of two or more of them for obtaining desired wavelength characteristics. Further, the compounds represented by general formula (I) may be used in combination with compounds other than these compounds.

The recording layers can contain various fading inhibitors for further improving the light resistance of the recording layers. The fading inhibitors include organic oxidizing agents and singlet oxygen quenchers. As the organic oxidizing agents used as the fading inhibitors, there are preferably used compounds described in Japanese Patent Laid-Open No. 151861/1998. The singlet oxygen quenchers include metal complexes, diimmonium salts and aminium salts represented by general formula (III), (IV) or (V) described in Japanese Patent Laid-Open No. 224793/1991.

Then, the structure of the information recording media of the invention will be illustrated. As described above, there is no particular limitation on the optical information recording medium of the invention, as long as it has the substrate having provided thereon the recording layer containing at least one compound represented by general formula (I). However, when the optical information recording medium of the invention is applied as the CD-R type medium, it is preferred that the recording layer containing at least one compound represented by general formula (I), a light reflective layer and a protective layer are provided in this order on a transparent disk-shaped substrate having a thickness of 1.2 ± 0.2 mm on which pre-grooves are formed at a track pitch of 1.4 µm to 1.8 µm. Further, when the optical information recording medium of the invention is applied as the DVD-R type medium, the following embodiments are preferred:
(1) An information recording medium having a thickness of 1.2 ± 0.2 mm comprising two laminates each comprising a transparent disk-shaped substrate having provided thereon the recording layer containing at least one compound represented by general formula (I) of the invention, each substrate having a diameter of 120 ± 0.3 mm or 80 ± 3 mm and a thickness of 0.6 ± 0.1 mm, pre-grooves being formed on the substrate at a track pitch of 0.6 µm to 0.9 µm, the recording layer being provided on the side where the pre-grooves are formed, the laminates being adhered to each other, with rendering the respective recording layer inside relative to the respective substrate; and
(2) An information recording medium having a thickness of 1.2 ± 0.2 mm comprising: a laminate comprising a transparent disk-shaped substrate having provided thereon the recording layer containing at least one compound represented by general formula (I) of the invention; and a disk-shaped protective plate having a size similar to that of the disk-shaped substrate, the substrate having a diameter of 120 ± 0.3 mm or 80 ± 3 mm and a thickness of 0.6 ± 0.1 mm, pre-grooves being formed on the substrate at a track pitch of 0.6 µm to 0.9 µm, the recording layer being provided on the side where the pre-grooves are formed, the laminate being adhered to the protective plate, with rendering the recording layer inside relative to the substrate.

Also in the above-mentioned embodiments, it is preferred that a reflective layer is provided on the recording layer. Further, a protective layer may be provided on the'reflective layer.

Methods for producing the information recording media of the invention will be described below.

For the DVD-R type recording media, the information recording media of the invention can be produced basically employing the materials used in the production of the CD-R type information recording media with the exception that substrates are used on which pre-grooves are formed at a narrower track pitch than those of the CD-R type media for attaining a higher recording density. That is to say, the DVD-R type information recording medium can be produced by preparing two laminates in each of which the recording layer, the light reflective layer, and further optionally the protective layer are formed on the substrate in this order, and adhering these two laminates to each other, or by adhering the laminate to the disk-shaped protective substrate having a size approximately similar to that of the substrate of the laminate, each using an adhesive with rendering the recording layer(s) inside relative to the substrate(s).

The information recording media of the invention can be produced, for example, by the following methods. The substrates (including the protective substrates) can be arbitrarily selected from various materials used as the substrates of the conventional information recording media. The materials for the substrates include, for example, glass; polycarbonates; acrylic resins such as polymethyl methacrylate; vinyl chloride resins such as polyvinyl chloride and vinyl chloride copolymers; epoxy resins; amorphous polyolefins; and polyesters. They may be used in combination as so desired. These materials can be used in the film form or in the rigid substrate form. Of the above-mentioned materials, polycarbonates are preferred in terms of moisture resistance, dimensional stability and cost.

Undercoat layers may be provided on surfaces of the substrates on which the recording layers are formed, for improving smoothness, enhancing adhesion and preventing deterioration of the recording layers. Materials for the undercoat layers include, for example, polymers such as polymethyl methacrylate, acrylic acid-methacrylic acid copolymers, styrene-maleic anhydride copolymers, polyvinyl alcohol, N-methylolacrylamide, styrene-vinyltoluene copolymers, chlorosulfonated polyethylene, nitrocellulose, polyvinyl chloride, chlorinated polyolefins, polyesters, polyimides, vinyl acetate-vinyl chloride copolymers, ethylene-vinyl acetate copolymers, polyethylene, polypropylene and polycarbonates; and surface modifiers such as silane coupling agents. The undercoat layers can be formed by dissolving or dispersing the above-mentioned materials in appropriate solvents to prepare coating solutions, and applying the resulting coating solutions to the surfaces of the substrates by coating methods such as spin coating, dip coating and extrusion coating. The thickness of the undercoat layers is generally within the range of 0.005 µm to 20 µm, and preferably within the range of 0.01 µm to 10 µm.

Grooves for tracking or unevenness (pre-grooves) for expressing information such as address signals are usually formed on the substrates (or undercoat layers). It is preferred that these pre-grooves are directly formed on the substrates at the above-mentioned track pitch in injection molding or extrusion molding of the resin materials such as polycarbonates. The pre-grooves may be formed by providing a pre-groove layer. As a material for the pre-groove layer, there can be used a mixture of at least one monomer (or oligomer) selected from a monoester, a diester, a triester and a tetraester of acrylic acid, and a photopolymerization initiator. The pre-groove layer is formed, for example, by applying a mixed solution of at least one of the above-mentioned acrylic acid esters and the photopolymerization initiator onto a mother die (stamper) precisely fabricated, further placing the substrate on the resulting coated layer, then irradiating the coated layer with ultraviolet rays through the substrate or the mother die, thereby hardening the coated layer to fixedly adhere the coated layer to the substrate, and thereafter separating the substrate from the mother die. The thickness of the pre-groove layer is generally within the range of 0.05 µm to 100 µm, and preferably within the range of 0.1 µm to 50 µm.

The depth of the pre-grooves preferably ranges from 300 to 2000 Å, and the half-width thereof preferably ranges from 0.2 µm to 0.9 µm. The use of the pre-grooves having a depth ranging from 1500 to 2000 Å can improve the sensitivity without a substantial reduction in reflectance, which is particularly advantageous to the CD-R type recording media.

The recording layers comprising the dye compounds represented by the above-mentioned formula according to the invention are provided on the surfaces of the substrates (or undercoating layers) on which the pre-grooves are formed.

The recording layers can be formed by dissolving the dyes according to the invention, and further optionally the quenchers and binders in solvents to prepare coating solutions, and then, applying the coating solutions onto the surfaces of the substrates to form coating films, followed by drying. Solvents for the coating solutions for the formation of the dye recording layers include esters such as methyl lactate, ethyl lactate, butyl acetate and cellosolve acetate; ketones such as methyl ethyl ketone, cyclohexanone and methyl isobutyl ketone; chlorinated hydrocarbons such as dichloromethane, 1,2-di-chloroethane and chloroform; amides such as dimethylformamide; hydrocarbons such as cyclohexane; ethers such as tetrahydrofuran, ethyl ether and dioxane; alcohols such as ethanol, n-propanol, isopropanol, n-butanol and diacetone alcohol; fluorine solvents such as 2,2,3,3-tetrafluoropropanol; and glycol ethers such as ethylene glycol monomethyl ether, ethylene glycol monoethyl ether and propylene glycol monomethyl ether. The above-mentioned solvents can be used alone or as a combination of two or more of them, considering the solubility of the dyes to be used. Various additives such as antioxidants, UV absorbers, plasticizers and lubricants may further added to the coating solutions, depending on their purpose.

Examples of binders include natural organic polymers such as gelatin, cellulose derivatives, dextran, rosin and rubber; and synthetic organic polymers such as hydrocarbon resins such as polyethylene, polypropylene, polystyrene and polyisobutylene; vinyl resins such as polyvinyl chloride, polyvinylidene chloride and vinyl chloride-vinyl acetate copolymers; acrylic resins such as polymethyl acrylate and polymethyl methacrylate; polyvinyl alcohol; chlorinated polyethylene; epoxy resins; butyral resins; rubber derivatives; and initial condensation products of thermosetting resins such as phenol-formaldehyde resins. When the binder is used as the material for the recording layer in combination with the dye, generally, a 0.01-fold to 50-fold excess of binder (by weight ratio), preferably, a 0.1-fold to 5-fold excess of binder (by weight ratio), in relation to the amount of dye, is used. The concentration of the coating solutions thus prepared is generally within the range of 0.01% to 10% by weight, and preferably within the range of 0.1% to 5% by weight.

Coating methods include spraying, spin coating, dip coating, roll coating, blade coating, doctor roll coating and screen printing. The recording layers may be either monolayers or multilayers. The thickness of the recording layers is generally within the range of 20 nm to 500 nm, and preferably within the range of 50 nm to 300 nm.

The reflective layers are provided on the recording layers for improving the reflectance in the reproduction of information. Light reflective materials, materials for the reflective layers, are materials having high reflectance to the laser beams, and examples thereof include metals such as Mg, Se, Y, Ti, Zr, Hf, V, Nb, Ta, Cr, Mo, W, Mn, Re, Fe, Co, Ni, Ru, Rh, Pd, Ir, Pt, Cu, Ag, Au, Zn, Cd, Al, Ga, In, Si, Ge, Te, Pb, Po, Sn and Bi, semi-metals and stainless steel. Of these, preferred are Cr, Ni, Pt, Cu, Ag, Au, Al and stainless steel. They may be used either alone or as a combination of two or more of them. Further, they may also be used as alloys. The reflective layers can be formed on the recording layers, for example, by vapor deposition, sputtering or ion plating of the above-mentioned reflective materials. The thickness of the reflective layers is generally within the range of 10 nm to 300 nm, and preferably within the range of 50 nm to 200 nm.

On the reflective layers, the protective layers may be provided for protecting the recording layers physically and chemically. The protective layers may also be provided on the side where no recording layers are provided, for enhancing scratch resistance and moisture resistance. Examples of materials used in the protective layers include inorganic materials such as SiO, SiO₂, MgF₂, SnO₂ and Si₃N₄, and organic materials such as thermoplastic resins, thermosetting resins and UV-curing resins. The protective layers can be formed, for example, by laminating the reflective layers and/or the substrates with films obtained by extrusion of plastics through adhesives. Alternatively, they may be provided by methods such as vacuum vapor deposition, sputtering and coating. In the case of the thermoplastic resins or the thermosetting resins, the protective layers can also be formed by dissolving these resins in appropriate solvents to prepare coating solutions, and then, applying the resulting coating solutions, followed by drying. In the case of the UV-curing resins, the resins can be applied as such, or dissolved in appropriate solvents to prepare coating solutions, which are applied, followed by irradiation of UV light to cure the resins, thereby forming the protective layers. Various additives such as antistatic agents, antioxidants and UV absorbers may be added to these coating solutions depending on their purpose. The thickness of the protective layers is generally within the range of 0.1 µm to 100 µm.

According to the above-mentioned processes, the laminates comprising the substrates having provided thereon the recording layers, the light reflective layers and the protective layers can be produced. The DVD-R type information recording medium having two recording layers can be produced by adhering the two laminates produced as described above to each other, with rendering the respective recording layer inside relative to the respective substrate. Methods for adhering the laminates include a method using a slow-acting ultraviolet curing adhesive, a hot melt method and a method of adhering the laminates with an adhesive tape. From the viewpoints of damages to the recording layers and cost, the method using the slow-acting ultraviolet curing adhesive is preferred. As the adhesive, a solventless adhesive is preferably used. Coating methods of the adhesives include spraying, spin control coating, roll coating and screen printing. Of these, screen printing is preferred. Further, the DVD-R type information recording medium having a recording layer only on one side can be produced by adhering the laminate to the disk-shaped protective substrate having a size approximately similar to that of the substrate of the laminate with the adhesive, with rendering the recording layer inside relative to the substrate.

The information recording method of the invention is conducted using the above-mentioned information recording medium, for example, in the following manner.

First, the information recording medium is irradiated from the substrate side thereof with light for recording such as a semiconductor laser beam, while rotating the medium at a constant linear speed (1.2 m/second to 14 m/second for the CD format) or at a constant angular speed. It is considered that this light irradiation results in the formation of voids in the interface of the recording layer and the reflective layer (the voids are formed, accompanying the deformation of the recording layer or the reflective layer, or the deformation of both layers), or in the building-up deformation of the substrate, or in changes in the index of refraction due to the discoloration of the recording layer or changes in the association state, which causes recording of information. As the recording light, there is used a laser beam in the visible light region, that is to say, in the case of the CD-R type recording medium, a semiconductor laser beam having a center oscillation wavelength of 780 nm. In the case of the DVD-R type recording medium, a laser beam having an oscillation wavelength ranging from 600 nm to 700 nm (preferably from 620 nm to 680 nm, and more preferably fro 630 nm to 660 nm) is generally used. The information recorded as described above can be reproduced by irradiating the information recording media from the substrate side thereof with a semiconductor laser beam having the same wavelength as the laser beam used in recording, while rotating the media at the same constant linear speed as described above, and detecting the reflective light thereof.

### EXAMPLES

The present invention will be illustrated in greater detail with reference to the following Examples, but the invention should not be construed as being limited thereto.

### EXAMPLE 1

A polycarbonate resin was formed into a 0.6-mm thick, 120-mm diameter substrate having spiral pre-grooves (depth: 150 nm, width: 290 nm, track pitch: 0.74 µm) by use of an injection molding machine (manufactured by Sumitomo Heavy Industries, Ltd. Oxonol dye compound (I-8) was dissolved in 100 ml of 2,2,3,3-tetrafluoropropanol to prepare a coating solution (dye concentration: 1.5% by weight). This coating solution was applied by spin coating onto a surface of the substrate on which the spiral pre-grooves were formed to form a dye layer. At this time, the thickness of the dye layer was 80 nm. Subsequently, silver was sputtered on the dye layer to form a reflective layer having a thickness of about 150 nm. Then, an ultraviolet curing resin (Daicure Clear SD-318, manufactured by Dainippon Ink & Chemicals, Inc.) was applied onto the reflective layer by spin coating, and irradiated with ultraviolet rays by use of a metal halide lamp to form a protective layer having a thickness of about 7 µm, thus obtaining a 0.6-mm thick disk A. On the other hand, silver was sputtered on the above-mentioned substrate without coating with the dye coating solution, and the protective layer was formed thereon to form a 0.6-mm thick disk B having no dye recording layer. The disk A and the disk B were adhered to each other to form one disk in the following manner. First, a slow-acting cationic polymerization type adhesive (SK 7000, manufactured by Sony Chemicals Corp.) was applied onto the protective layers of the disk A and the disk B by screen printing. The mesh size of a screen printing plate used at this time was 300 meshes. Then, immediately after irradiation with ultraviolet rays by use of a metal halide lamp, the disk A and the disk B were adhered to each other, facing the respective protective layers inside, and pressed from both sides. After standing for about 5 minutes, the adhesive was completely cured to complete one disk having a thickness of 1.2 mm.

### EXAMPLES 2 TO 8 AND COMPARATIVE EXAMPLES 1 TO 4

DVD-R type disks of Examples 2 to 8 and Comparative Examples 1 to 4 were obtained in the same manner as in Example 1, with the exception that the above-mentioned compound (I-8) was replaced with compounds shown in Table 1.

### Evaluation as Optical Disk

A signal was recorded on each of the DVD-R type optical disks of Examples and Comparative Examples with a DDU 1000 evaluating device (manufactured by Balstec Co.) using a laser beam having a wavelength of 655 nm (pick up to Na 0.6), a constant line speed of 3.49 m/s, a modulation frequency of 4 MHz and an optimum recording power within the range of 6 mW to 14 mW. Then, using a laser beam having the same wavelength as the recording laser beam, the signal was reproduced at a laser power of 0.5 mW, and the modulation degree of 14T and the jitter were measured.

### Resistance against Heat and Humidity

Each sample recorded as described above were stored for 24 hours under circumstances of a high temperature of 80°C and a high humidity of 85%, and the modulation degree and the jitter were measured in the same manner as described above.

Evaluation results obtained are shown in Table 1.

**TABLE 1**

| | Compound Used in Recording Layer | Recording Reproduction Characteristics (before storage) | | Recording Reproduction Characteristics (after storage at 80°C, 85%) | |
|---|---|---|---|---|---|
| | | 14T | Jitter | 14T | Jitter |
| Example 1 | (I-8) | 70 | 6.9 | 67 | 7.4 |
| Example 2 | (I-10) | 71 | 7.4 | 69 | 7.5 |
| Example 3 | (I-15) | 68 | 7.8 | 62 | 8.5 |
| Example 4 | (I-52) | 65 | 8.2 | 55 | 9.9 |
| Example 5 | (I-72) | 67 | 8.2 | 61 | 8.9 |
| Example 6 | (I-80) | 74 | 7.6 | 68 | 9.4 |
| Example 7 | (I-87) | 74 | 7.6 | 68 | 9.1 |
| Example 8 | (I-94) | 59 | 7.2 | 55 | 8.0 |
| Comparative Example 1 | Dye (1) for Comparison | 70 | 10.5 | 42 | Impossible to measure |
| Comparative Example 2 | Dye (2) for Comparison | 59 | 9.2 | 40 | Impossible to measure |
| Comparative Example 3 | Dye (3) for Comparison | 71 | 7.4 | 50 | 14.7 |
| Comparative Example 4 | Dye (4) for Comparison | 70 | 7.1 | 52 | 13.5 |

### Dyes for Comparison Used in Comparative Examples 1 to 4

Dye (1) for Comparison: Specific example (32) described in Japanese Patent Laid-Open No. 2000-52658

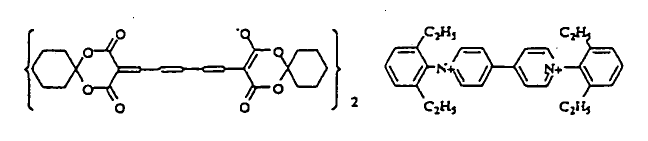

Dye (2) for Comparison: Specific example (79) described in Japanese Patent Laid-Open No. 2000-52658

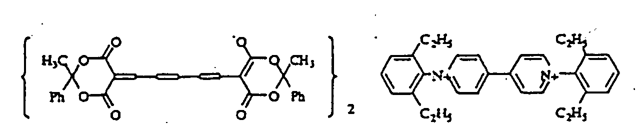

Dye (3) for Comparison: Specific example (106) described in Japanese Patent Laid-Open No. 2000-52658

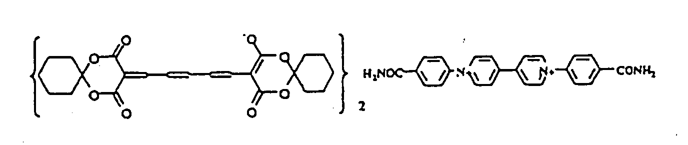

Dye (4) for Comparison: Specific example (107) described in Japanese Patent Laid-Open No. 2000-52658

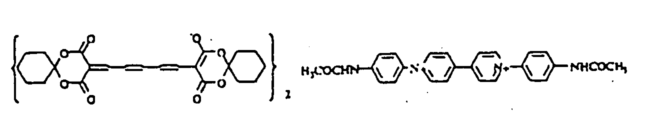

The results shown in Table 1 reveal that the DVD-R type optical disks in which each recording layer contains at least one compound represented by general formula (I) according to the invention (Examples 1 to 8) indicate high modulation degree and give low jitter, which shows that excellent recording and reproduction characteristics are obtained. Further, compared with the disks for comparison (Comparative Examples 1 to 4), the disks of the invention maintain good recording and reproduction characteristics even after execution of the heat and humidity resistance test, which shows that the disks of the invention are excellent in heat and humidity resistance.

According to the invention, there are provided the write once-type optical information recording media excellent in recording and reproduction characteristics and keeping quality, in which recording and reproduction of information can be performed by laser beam irradiation. Further, there are provided the optical information recording methods using the optical information recording media, giving excellent recording and reproduction characteristics and keeping quality.

While the invention has been described in detail and with reference to specific examples thereof, it will be apparent to one skilled in the art that various changes and modifications can be made therein without departing from the spirit and scope thereof.

Preferred embodiments are:
1. An oxonol dye compound represented by the following general formula (I): 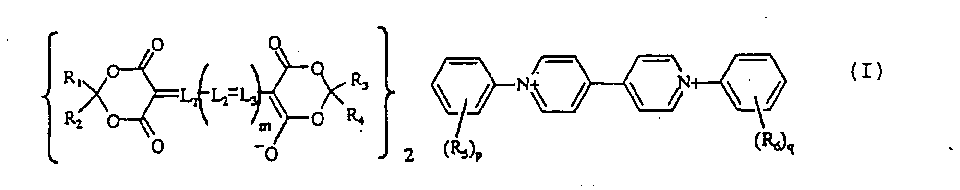 wherein R₁, R₂, R₃ and R₄ each independently represents an hydrogen atom, an alkyl group, an aryl group, an aralkyl group or a heterocyclic group; L₁, L₂ and L₃ each independently represents a methine group which may have a substituent group; m represents an integer of 0 to 3; R₅ and R₆ each represents an alkyl group having 4 to 8 carbon atoms, an aryl group having 6 to 12 carbon atoms, an aralkyl group having 7 to 12 carbon atoms, a chlorine atom, a bromine atom, a hydroxyl group, an acyl group having 7 to 12 carbon atoms, an aryloxy group having 6 to 12 carbon atoms or an amido group having 4 to 8 carbon atoms; p and q each represents an integer of 1 to 3; R₁ and R₂, and R₃ and R₄ may combine with each other to form a ring; when m is 2 or more, plural -L₂=L₃- groups may be the same or different; and when p and q are integers of 2 or more, plural R₅ and R₆ groups may be the same or different, respectively.
2. An optical information recording medium comprising a substrate having thereon a recording layer capable of recording information by irradiation of a laser beam, wherein said recording layer contains an oxonol dye compound represented by general formula (I) according to claim 1.
3. The optical information recording medium according to claim 2, further comprising at least one of a reflective layer and a protective layer.
4. An optical information recording method comprising irradiating an optical information recording medium with a laser beam having a wavelength of 630 nm to 660 nm to record information thereon, wherein the optical information recording medium is according to claim 2 or 3.

## Claims

1. A dye compound represented by the following general formula (I): 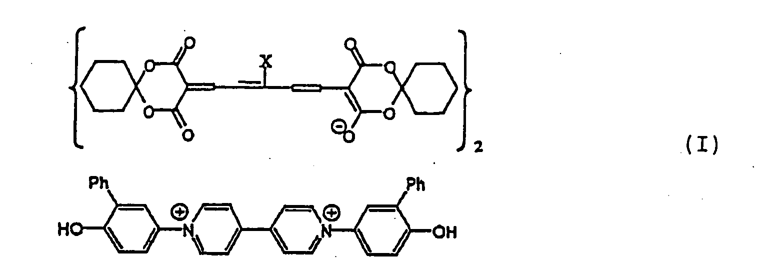 wherein X represents a hydrogen atom or a phenyl group.

2. An optical information recording medium comprising the dye compound according to claim 1.

3. A process for synthesizing the dye compound according to claim 1, which comprises the steps of:
synthesizing a dye compound represented by general formula (II) shown below from compound G shown below: 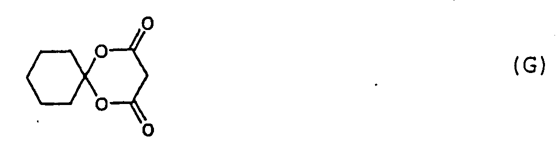 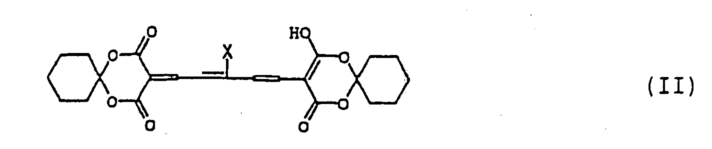
wherein X represents a hydrogen atom or a phenyl group; and synthesizing the dye compound represented by general formula (I) from the compound represented by general formula (II).

4. An optical information recording medium comprising a dye compound synthesized by the process according to claim 3.

5. A coating solution comprising 2,2,3,3-tetrafluoropropanol and the dye compound according to claim 1 dissolved therein.

6. An optical information recording medium obtained by applying the coating according to claim 5 onto a transparent substrate.

7. Use of the optical information recording medium according to claim 2 for information recording or reading with a laser beam having wavelength of from 600 to 700 nm.

8. A method for optical information recording or reading comprising irradiating an optical information recording medium with a laser beam having wavelength of from 600 to 700 nm to record or read information thereon,
wherein the optical information recording medium is according to claim 2.
